# EUROPEAN PATENT APPLICATION

(11) **EP 4 450 041 A1**
(43) Date of publication of application: **23.10.2024**
(21) Application number: 23168319.4
(22) Date of filing: 17.04.2023
(51) Int. Cl.: A61F 13/537, B32B 5/26, D04H 1/492, D04H 1/498

(54) **ACQUISITION AND DISTRIBUTION COMPOSITE, ABSORBENT HYGIENE ARTICLE AND METHOD TO PRODUCE**

(71) Applicant: Nitto Advanced Film Gronau GmbH, 48599 Gronau (DE); NITTO DENKO CORPORATION, Ibaraki-shi Osaka 567-8680 (JP)
(72) Inventor: Trinkaus, Jan Michael, 53881 Euskirchen (DE); Rodriguez, Patricia, 06449 Aschersleben (DE)
(74) Representative: Andrejewski - Honke Patent- und Rechtsanwälte Partnerschaft mbB

(57) **Abstract**

The invention relates to an acquisition and distribution composite (2) with a liquid acquisition side (5) comprising first nonwoven fibers (7), a hydrophilic core (9) and liquid delivery side (6) comprising second nonwoven (8) fibers. According to the invention an asymmetric structure with the linear density measured in dtex of the first nonwoven fibers (7) is at least 0.7 dtex greater than that of the second nonwoven fibers (8). In addition an absorbent hygiene article comprising said acquisition and distribution composite (2) and a method to produce an acquisition and distribution composite (2).

## Description

The present invention relates to an acquisition and distribution composite with a liquid acquisition side comprising first nonwoven fibers, a hydrophilic core and a liquid delivery side comprising second nonwoven fibers. The invention furthermore relates to an absorbent hygiene article comprising said acquisition and distribution composite and a method to produce said acquisition and distribution composite.

Absorbent hygiene articles such as disposable diapers, sanitary napkins and panty liners, are usually composed of a liquid-permeable topsheet, a liquid-impermeable backsheet and an absorbent core in between. In many applications the liquid to be absorbed like urine or blood occurs local and/or in the short period of time so that without distribution of the liquid an insufficient absorption or a local overload of the absorbent core may occur.

Considering baby diapers or adult incontinence pants, high, localized loads in some areas, and very low loads in others, depending on the wear situation of the wearer (on belly, on back, standing, sitting) are observed. Consequently, the diaper or pant leaks when the capacity is locally saturated, or the diaper or pant sags, when the weight of the load pulls it down on the wearer, leading to bad fit, and leakage.

To allow a certain distribution of the liquid absorbent hygiene products can comprise an acquisition and distribution materials (ADL) above the absorbent core to move bodily liquids quickly away from the surface, into the permanently absorbent core. Such an acquisition and distribution material can be directly provided as topsheet or as a secondary topsheet below a permeable primary topsheet.

The aim is not only to transport the liquid rapidly in the direction of the thickness, but also to distribute it over a large area, whereby transport against the force of gravity by capillary forces can also be useful.

Acquisition and distribution composites are known from practice, which are formed starting from several nonwoven and/or cellulose layers by air-through bonding or by adhesive.

Generic acquisition and distribution composites are known from EP 3 143 976 A1 and WO 2022/016051 A1. According to this prior art a hydrophilic core of short cellulose fibers is provided between a first nonwoven layer and a second nonwoven layer, wherein the layers are consolidated by hydroentanglement, i.e. this stream of water jets acting on the material.

By the hydroentanglement the fibers of the first nonwoven layer, the fibers of the second nonwoven layer and the short cellulose fibers are distributed in the material so that the layers interpenetrate each other to a certain extend. Nevertheless the fibers of the first nonwoven are mainly situated on side of the composite and the fibers of the second nonwoven are mainly situated on opposite of the composite with the short cellulose fibers in between. Especially the fine short cellulose fibers are also distributed in the nonwoven layers and can thus enhance the capability of the liquid transport in the direction of thickness.

The invention is based on the problem of providing an acquisition and distribution composite with improved performance properties. In particular, a good liquid absorption and distribution shall be achieved. Furthermore, an absorbent hygiene article and a method for manufacturing the acquisition and distribution composite are to be disclosed.

The object of the invention and the solution to the problem are an absorption and distribution composite according to claim 1, an absorbent hygiene article according to claim 18, and a process for producing an absorption and distribution composite according to claim 20.

Starting from a generic absorption and distribution composite, according to a first aspect of the invention, an asymmetric structure is thus provided, wherein linear density measured in dtex of the first nonwoven fibers is at least 0.7 dtex, preferably at least 1 dtex greater than that of the second nonwoven fibers.

In the context of the invention, an acquisition and distribution composite asymmetric in the thickness direction is provided, with one side defined as the liquid acquisition side and the opposite side defined as the liquid delivery side. The liquid acquisition side is coarser due to the greater linear density of the first nonwoven fibers, resulting in comparatively large pores and only comparatively small capillary forces between the fibers. Liquid can thus easily enter and reach the hydrophilic core, preferably comprising pulp fibers.

The liquid delivery side, on the other hand, is finer, so that certain capillary forces can also be effective to a greater extent for the onward transfer of the liquid.

In this context, the invention is based on the realization that the relevant mechanisms on the two sides of the acquisition and distribution composite are fundamentally different.

On the liquid acquisition side, a large amount of liquid must be absorbed when required, but without a direct supply of liquid, the aim is to achieve as dry a sensation as possible. On the liquid delivery side, the amount of liquid occurring locally is less due to the distribution of liquid in the hydrophilic core, but the liquid must then be uniformly removed when the hydrophilic core becomes somewhat saturated or supersaturated. It can also be an advantage that there are smaller pores compared to the liquid acquisition side, whereby a certain resistance is also achieved for the passage of liquid, so that it is initially distributed in a suitable manner in the hydrophilic core. In the case of the liquid delivery side, moderate permeability is thus particularly advantageous in the presence of liquid transport supported by capillary forces.

According to a preferred embodiment, the acquisition and distribution composite is hydroentangled. Hydroentangling provides a consolidation of the composite by the entanglement of the fibers, both of the same type and of different types, which makes it possible to link individual layers together and to increase the overall strength of the composite. Thus fibers initially provides as separate layer are distributed in the thickness of the composite to form to a certain extend interpenetrating layers.

When the hydrophilic core is provided with comparable soft and short pulp fibers the hydrophilic core can be stabilized by the first and second nonwoven fibers. Furthermore a fraction of the pulp fibers are also distributed to the liquid acquisition side and the liquid delivery side and thus modulation the capabilities for the transport of liquid. Finally hydroentangling compress the structure to a defined thickness, which corresponds to a defined average pore size for a given composition.

Particularly preferred hydroentangling is provided on smooth surfaces with waterjets from both sides of the acquisition and distribution composite to achieve a advantageous fiber distribution.

The composite can solely be consolidated by hydroentangling without any further bonding, consolidation or structuring. Especially when the hydroentangling is provided on smooth surfaces the composite is easy to manufacture and comprises in a scale above the coarseness due to the diameter and density of the fibers a homogenous structure in plane. As smooth surfaces smooth drums can be provided as an example, wherein only small perforations for the passage of water are provided which cannot contribute to an embossing or a comparable deformation of the composite.

The acquisition and distribution composite basis weight is preferably between 50 g/cm² and 160 g/cm² (gsm - grams per square centimeter). The acquisition and distribution composite is usually provides for single-use product and especially single-use absorbent hygiene article like diapers, sanitary napkins, panty liners and adult incontinence pants so that low material input, low manufacturing costs and optionally also biodegradability are to be aimed for.

Furthermore, the person skilled in the art can select a suitable basis weight, taking into account the specific application and the amount and type of liquid involved.

In the case of a sanitary napkin, for example, small quantities of liquid are produced over a longer period of time, whereby particular importance is also given to a high level of wearer comfort. Accordingly, a comparatively low basis weight in the specified range or below is appropriate.

In the case of an adult incontinence pant, on the other hand, very large quantities of liquid may occur over a short period of time, so that a comparatively large basis weight in the specified range or above may be appropriate.

As a rule, the buffering and distribution capabilities of the acquisition and distribution composite increase with increasing basis weight.

The first nonwoven fibers may comprise a linear density ("fineness") of 2 dtex to 8 dtex, preferably 3 dtex to 7 dtex and the second nonwoven fibers may comprise a linear density of 0.7 dtex to 4 dtex, preferably 1.5 dtex to 3.3 dtex.

Although the specified parameter ranges overlap at their limits, another criterion that must always be taken into account is that the linear density of the first nonwoven fibers is at least 0.7 dtex greater than that of the second nonwoven fibers. For example, if the second nonwoven fibers have a linear density of 4 dtex, the first nonwoven fibers have at least a linear density of 4.7 dtex. If the first nonwoven fibers have a linear density of 2 dtex, the second nonwoven fibers have a linear density of at most 1.3 dtex.

The length of the first nonwoven fibers can be in the range between 20 mm to 80 mm and the length of the second nonwoven fibers can be in the range between 10 mm and 50 mm.

The composite may be formed from 20 to 50 weight percent of the first nonwoven fibers and/or from 20 to 50 weight percent of the second nonwoven fibers. The amount of short cellulose fibers, especially pulp fibers may the in the range from 10 to 50 weight percent.

The weight percentage of the first nonwoven fibers, the second nonwoven fibers and the short cellulose fibers may sum up to 90 weight percent, preferably 95 weight percent and most preferred 100 weight percent. In the last case the acquisition and distribution composite completely consists of first nonwoven fibers, the second nonwoven fibers and the short cellulose fibers. The short cellulose fibers are short in comparison to the first and second nonwoven fibers and might have a typical length between 0.5 mm and 6 mm, preferably 1 mm to 5 mm, especially between 2 mm and 3 mm.

In the context of the invention, cellulose fibers are understood to be, according to common understanding, fibers consisting essentially of cellulose, i.e. cellulose chains. In practice, natural cellulose fibers and manufactured cellulose fibers are known. Comparable short manufactured cellulose fibers can be obtained by mechanical and/or chemical decomposition of wood and other plant materials, in particular by removal of lignin. Comparable long manufactured cellulose fibers can be obtained as regenerated cellulose in a spinning process.

In order to distinguish between the different types of cellulose fibers, the invention distinguishes between short cellulose fibers with a length of less than 10 mm and in particular less than 5 mm and long cellulose fibers with a length of more than 10 mm and in particular more than 20 mm. Short cellulose fibers are usually obtained as pulp directly by disintegrating shredded wood or other plant-based materials. Long cellulose fibers, on the other hand, are usually present as natural fibers such as cotton or as regenerated fibers, which are generally referred to as viscose or rayon. Depending on the starting material and manufacturing process, names such as Lyocell and Modal are also known.

The short cellulose fibers might also be at least partially cross-linked to enhance the bulkiness. Cross-linked short cellulose fibers are for example disclosed in US 5 137 537 and US 5 998 511.

Preferably, only one type of fiber is provided for each of the first nonwoven fibers and the second nonwoven fibers. In principle, the first nonwoven fibers and/or the second nonwoven fibers can each also be formed, for example, by a first fiber type and a second fiber type. Preferably, it is also provided that previously defined criterion of different linear density between the first nonwoven fibers and the second nonwoven fibers is always fulfilled when considering all fiber types.

The first nonwoven fibers and/or the second nonwoven fibers can be provided as carded nonwoven each. For the first nonwoven fibers and/or the second nonwoven fibers a polyester or long cellulose fibers can be provided as main material component. Long cellulose fibers can be provided as natural fibers like cotton or as regenerated cellulose fibers, especially viscose.

Especially the first nonwoven fibers and the second nonwoven fibers can advantageous be provided as separate nonwoven webs, especially carded nonwovens, of the same material or at least with the same main material component. For example the first nonwoven fibers and the second nonwoven fibers can comprise polyester like polyethylenterephthalat (PET) as main material component.

As an alternative the first nonwoven fibers and the second nonwoven fibers can consist of a biodegradable material as natural fibers or natural based fibers like cotton or viscose. Considering short cellulose fibers (pulp) as preferred material for the hydrophilic core, the complete acquisition and distribution composite can be biodegradable. Accordingly the acquisition and distribution composite can be combined with other biodegradable components to form a complete biodegradable absorbent hygiene article. Such a biodegradable absorbent hygiene article can be composted wherein even accumulated body liquids and excretions can be handled and biologically decomposed under controlled condition. Even in case that such a biodegradable absorbent hygiene article is improperly discarded into the environment the material will decompose over a certain time period.

As mentioned above especially the first nonwoven fibers can also be formed by a first fiber type and a second fiber type. As an example an admixture of comparable stiff fibers like hemp fibers is conceivable to enhance the mechanical stability of the composite. Especially a blend of 60 wt% (weight percent) to 90wt% of viscose fibers and 10 wt% to 40 wt% hemp fibers can be suitable for as biodegradable material, especially provided as carded nonwoven.

The acquisition and distribution composite as a whole comprises hydrophilic properties to allow an effective acquisition and distribution of liquids and especially body liquids.

Nevertheless, it may be envisaged that just the first nonwoven fibers themselves have hydrophobic properties. As described previously, liquid transport on the liquid acquisition side preferably takes place through pores of the coarse material, so that the wetting properties of the individual fibers are then of secondary importance. Hydrophobic properties of the first nonwoven fibers can be used to ensure that no moisture or liquid remains on the first nonwoven fibers after liquid has passed through. It should also be noted that absorbent hygiene articles are usually intended to absorb liquid over a long period of time and are also frequently not changed immediately after an absorption. Thus, hydrophobic properties of the first nonwoven fibers can improve comfort for a wearer. For example, the risk of skin irritation and an unpleasant wet feeling can be avoided.

To provide the first nonwoven fibers a material may be chosen which comprises intrinsically hydrophobic properties. Furthermore specific admixtures or coating can be provided to achieve hydrophobic properties. The first nonwoven fibers may at least partially comprise a mantle that reduces wettability, so that especially the wettability of natural fibers or viscose can be reduced. The mantle can contain natural ingredients such as waxes or oils as well as chemically produced hydrophobic ingredients. A mantle of natural ingredients can be applied to the fibers. Alternatively, it is also known, for example, that cotton has a waxy skin when picked, and if treated gently, the waxy skin, which reduces wettability, remains on the fibers.

Suitable hydrophobic viscose fibers are marketed under the name Olea by Kelheim Fibers GmbH of Kelheim, Germany.

The invention also discloses an absorbent hygiene article comprising the acquisition and distribution composite described above, a usually liquid impermeable backsheet and an absorbent core between the backsheet and the acquisition and distribution composite.

The acquisition and distribution composite may be provided as topsheet of the absorbent hygiene article which can be in direct contact to a wearer. According to an alternative embodiment the acquisition and distribution composite is provided between the absorbent core and a permeable topsheet. In the latter case the acquisition and distribution composite is also referred to as secondary topsheet.

The invention also discloses a method to produce the above defined acquisition and distribution composite comprising:
providing a first carded nonwoven web of first nonwoven fibers,
providing a second carded nonwoven web of second nonwoven fibers,
providing an airlaid hydrophilic core comprising short cellulose fibers after providing one of the first and second carded nonwoven and before providing the other of the first and second carded nonwoven,
hydroentangeling the three layer structure formed by the hydrophilic core and the first and second carded nonwoven to form the acquisition and distribution composite,
wherein the linear density measured in dtex of the first nonwoven fibers is at least 0.7 dtex greater than that of the second nonwoven fibers.

The invention is explained below with reference to figures. It shows:
- Fig. 1: a typical construction of an absorbent hygiene article in a schematic structure;
- Fig. 2: a schematic illustration of an absorbent hygiene article in use,
- Fig. 3: an acquisition and distribution composite of the absorbent hygiene article
- Fig. 4: a method to produce the acquisition and distribution composite.

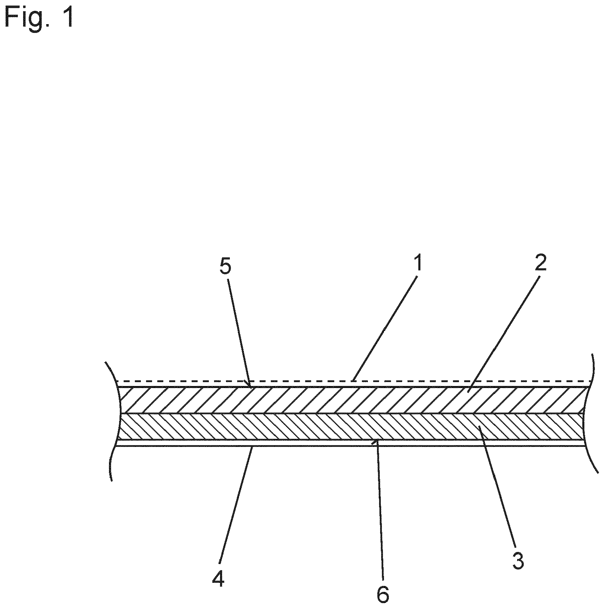

Fig.1 shows a typical construction of an absorbent hygiene article in a schematic structure comprising a permeable topsheet 1 being in a direct contact with a wearer in use, an acquisition and distribution composite 2, an absorbent core 3 and a liquid impermeable backsheet 4. The absorbent hygiene article can as an example be provided as diapers, sanitary napkins, panty liners and adult incontinence pants for a single use. In the shown embodiment the acquisition and distribution composite 2 can also be referred to as a secondary topsheet. As an alternative, the acquisition and distribution composite 2 might be provided as sole sheet, i.e. as sole topsheet.

In many applications the body liquid to be absorbed like urine or blood occurs local and/or in the short period of time so that the acquisition and distribution composite 2 distribute the liquid to avoid a local saturation of the absorbent core 3.

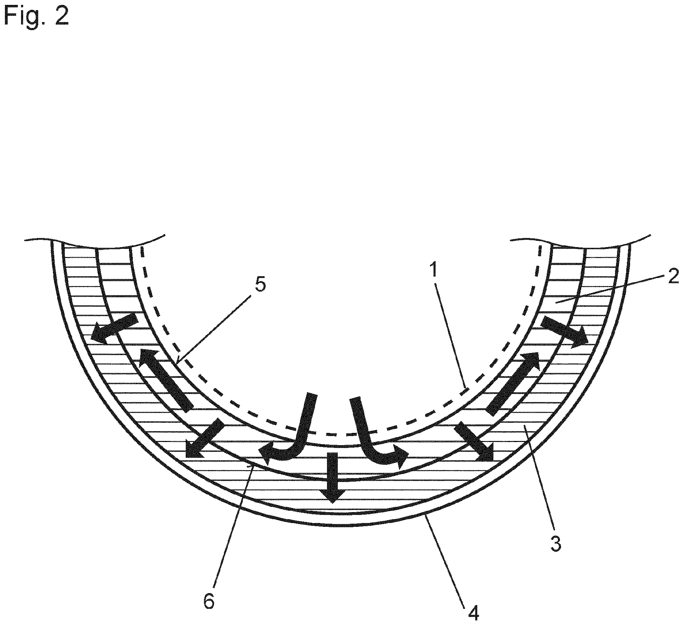

Fig. 2 shows a schematic illustration of an absorbent hygiene article like a diaper in use. For example urine usually occurs in a large amount in a low position of the diaper placed around the body of the wearer. Thus to allow a distribution of liquid also against gravitation the acquisition and distribution composite 2 exhibits capillary properties.

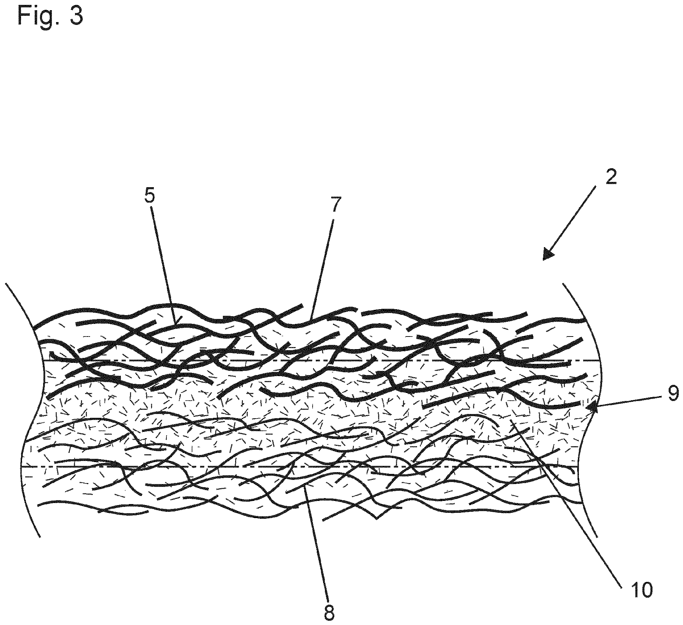

As shown in Fig. 3 the acquisition and distribution composite 2 comprises due to a hydroentanglement three interpenetrating layers. A coarse side of the acquisition and distribution composite 2 is orientated to the topsheet 1 and is thus provided as a liquid acquisition side 5. The finer opposite side of the acquisition and distribution composite 2 lies against the absorbent core 3 and is thus provided as a liquid delivery side 6.

The liquid acquisition side 5 comprises first nonwoven fibers 7 and liquid delivery side 6 comprises second nonwoven fibers 8, wherein an asymmetric structure is provided with the linear density measured in dtex of the first nonwoven fibers 7 is at least 0.7 dtex greater than that of the second nonwoven fibers 8. Between the liquid acquisition side 5 and the liquid delivery side 6 a hydrophilic core 9 of pulp fibers 10 is provided.

Due to the hydroentanglement the acquisition and distribution composite 2 is solidified and the fibers of the different layers are displaced to a certain extend. The first and second nonwoven fibers 7, 8 partially reach into the hydrophilic core 9 and contribute to an enhanced stability. The pulp fibers 10 are scatted to a certain extend in to the direction of the liquid acquisition side 5 and the liquid delivery side 6 whereby the liquid transport properties in direction of thickness are modulated. Nevertheless the structure of three layers is still clearly recognizable.

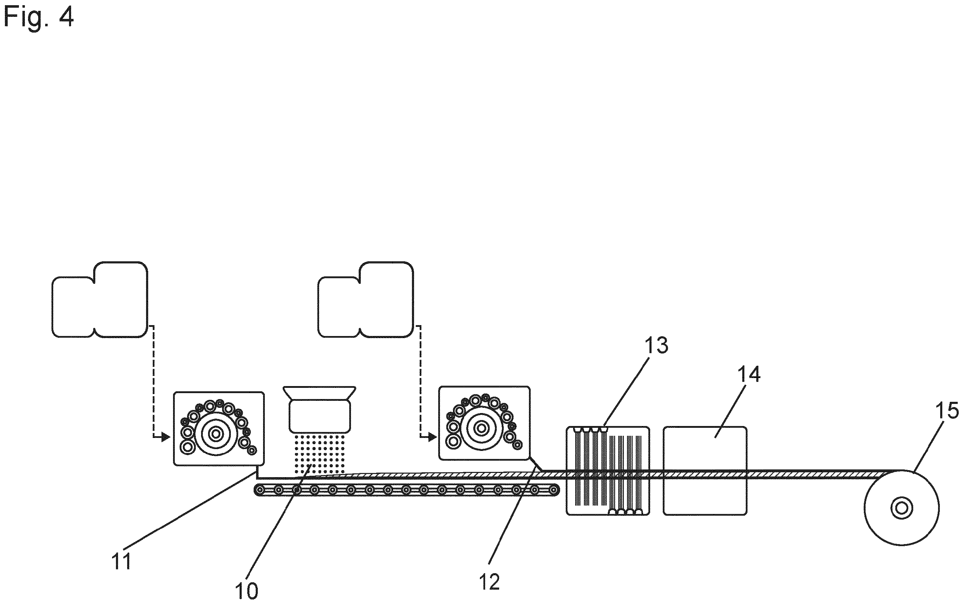

A method to produce the acquisition and distribution composite 2 is schematically shown in Fig. 4. At first the first nonwoven fibers 7 or the second nonwoven fibers 8 are provided as a first or second carded nonwoven 11, 12. By way of example, according to Fig. 4, the first carded nonwoven 11 consisting of the first nonwoven fibers 7 is deposited first, although a reverse arrangement is also possible.

Subsequently, the pulp fibers 10 are deposited as an airlaid layer on the first carded nonwoven 11 before the second carded nonwoven 12 consisting of the first nonwoven fibers 8 is applied. Than the three layer structure is fed to a hydroentanglement station 13 where the layers are consolidated with water jets from both sides.

The acquisition and distribution composite 2 achieved by hydroentanglement in finally dried in a drier 14 and wound on a roll 15. The roll 15 of the t he acquisition and distribution composite 2 can afterwards be transported and further processed for the production of absorbent hygiene article.

Various examples of embodiments according to the invention are shown below.

### Example 1:

An acquisition and distribution composite 2 was produced according to the above disclosed method with a layer structure shown in table 1. The acquisition and distribution composite 2 comprises a basis weight of 105 g/m² and comparable large pores layer at the liquid acquisition side 5. The acquisition and distribution composite 2 is especially suitable for diapers and allows the acquisition and distribution of large amount of suddenly occurring body liquids like urine. The acquisition and distribution composite 2 might be use as single topsheet of the diaper. The fibers at the liquid acquisition side 5 comprise an average length of 38 mm and the fibers at the liquid delivery side 6 comprise an average length of 60 mm. The pulp fibers 10 of the hydrophilic core 9 comprise an average length of about 2.7 mm ("Golden Isles Untreated Fluff - Grade 4881", GP Cellulose LLC, Atlanta, Georgia, US).

**table 1**

| layer arrangement | basis weight | layer type | fiber type | linear density |
|---|---|---|---|---|
| liquid acquisition side | 45 g/m² | carded | 100% PET | 10 dtex |
| hydrophilic core | 15 g/m² | airlaid | 100% pulp | |
| liquid delivery side | 45 g/m² | carded | 100% PET | 3.3 dtex |

### Example 2:

An acquisition and distribution composite 2 was produced according to the above disclosed method with a layer structure shown in table 2. The acquisition and distribution composite 2 comprises a basis weight of 60 g/m². The comparable light and thin acquisition and distribution composite 2 is especially suitable for female care products like sanitary napkins and allows the acquisition and distribution body liquids like blood. The acquisition and distribution composite 2 might be use as secondary topsheet below a primary topsheet 1 (see also fig. 1). The average length of the first nonwoven fibers 7 comprising a linear density of 3.3 dtex is about 60 mm and the average length of the second nonwoven fibers 8 comprising a linear density of 1.7 dtex is about 38 mm. For the hydrophilic core 9 the same pulp was used like in example 1.

**table 2**

| layer arrangement | basis weight | layer type | fiber type | linear density |
|---|---|---|---|---|
| liquid acquisition side | 20 g/m² | carded | 100% PET | 3.3 dtex |
| hydrophilic core | 25 g/m² | airlaid | 100% pulp | |
| liquid delivery side | 15 g/m² | carded | 100% PET | 1.7 dtex |

### Example 3:

An acquisition and distribution composite 2 was produced according to the above disclosed method with a layer structure shown in table 3. The acquisition and distribution composite 2 comprises a basis weight of 60 g/m². The comparable light and thin acquisition and distribution composite 2 is especially suitable for female care products like sanitary napkins and allows the acquisition and distribution body liquids like blood. The acquisition and distribution composite 2 might be use as secondary topsheet below a primary topsheet 1 (see also fig. 1). The complete acquisition and distribution composite 2 consist of biodegradable fibers, i.e. viscose and pulp. The acquisition and distribution composite 2 can thus be used to form a biodegradable absorbent hygiene article. The first nonwoven fibers 7 provided at the liquid acquisition 5 side consist of a hydrophobic viscose. Suitable hydrophobic viscose fibers are marketed under the name Olea by Kelheim Fibers GmbH of Kelheim, Germany. The hydrophobic properties of the first nonwoven fibers can improve comfort for a wearer. For example, the risk of skin irritation and an unpleasant wet feeling can be avoided. The fibers at the liquid acquisition side 5 comprise an average length of 50 mm and the fibers at the liquid delivery side 6 comprise an average length of 40 mm. For the hydrophilic core 9 the same pulp was used like in example 1

**table 3**

| layer arrangement | basis weight | layer type | fiber type | linear density |
|---|---|---|---|---|
| liquid acquisition side | 15 g/m² | carded | 100% hydrophobic viscose | 4.2 dtex |
| hydrophilic core | 25 g/m² | airlaid | 100% pulp | |
| liquid delivery side | 20 g/m² | carded | 100% Viscose | 1.7 dtex |

### Example 4:

An acquisition and distribution composite 2 was produced according to the above disclosed method with a layer structure shown in table 4. The acquisition and distribution composite 2 comprises a basis weight of 70 g/m². The acquisition and distribution composite 2 is especially suitable for female care products like sanitary napkins and allows the acquisition and distribution body liquids like blood. The acquisition and distribution composite 2 might be use as secondary topsheet below a primary topsheet 1 (see also fig. 1). The complete acquisition and distribution composite 2 consist of biodegradable fibers, i.e. viscose, hemp and pulp. The acquisition and distribution composite 2 can thus be used to form a biodegradable absorbent hygiene article. The first nonwoven fibers 7 provided at the liquid acquisition 5 side consist of a first fiber type of hydrophobic viscose with a weight share of 70% related to the corresponding first carded nonwoven 11 and hemp fibers with a weight share of 30%. The linear density of the hemp fibers as a natural product may vary but is usually higher or significantly higher than the linear density of the viscose fibers. For the hydrophilic core 9 the same pulp was used like in example 1

**table 4**

| layer arrangement | basis weight | layer type | fiber type | linear density |
|---|---|---|---|---|
| liquid acquisition side | 30 g/m² | carded | 70% hydrophobic viscose | 4.4 dtex (for viscose fibers) |
| | | | 30% hemp fibers | |
| hydrophilic core | 25 g/m² | airlaid | 100% pulp | |
| liquid delivery side | 15 g/m² | carded | 100% Viscose | 1.7 dtex |

### Example 5:

An acquisition and distribution composite 2 was produced according to the above disclosed method with a layer structure shown in table 5. The acquisition and distribution composite 2 comprises a basis weight of 100 g/m². The acquisition and distribution composite 2 is especially suitable for female care products like panty liners. The hydrophilic core comprises with 35 g/m² a comparable high basic weight. Thus the acquisition and distribution composite 2 comprises a significant integrated storage capacity and might even be used in an absorbent hygiene article for light use without an adjoining absorbent core 3. The acquisition and distribution composite 2 might be use as secondary topsheet below a primary topsheet 1 (see also fig. 1). For the hydrophilic core 9 the same pulp was used like in example 1.

**table 5**

| layer arrangement | basis weight | layer type | fiber type | linear density |
|---|---|---|---|---|
| liquid acquisition side | 38 g/m² | carded | 100% PET | 3.3 dtex |
| hydrophilic core | 35 g/m² | airlaid | 100% pulp | |
| liquid delivery side | 27 g/m² | carded | 100% PET | 1.7 dtex |

### Example 6:

An acquisition and distribution composite 2 was produced according to the above disclosed method with a layer structure shown in table 6. The acquisition and distribution composite 2 comprises a basis weight of 122 g/m² and comparable large pores layer at the liquid acquisition side 5. The acquisition and distribution composite 2 is especially suitable for diapers or light adult incontinence products like light adult incontinence pants and allows the acquisition and distribution of large amount of suddenly occurring body liquids like urine. The acquisition and distribution composite 2 might be use as single topsheet. The first nonwoven fibers 7 provided at the liquid acquisition 5 side consist of a first fiber type of PET fibers and a second fiber type of PET fibers with a weight share of 50% each. The first and second fiber type differ in the linear density and length with an average length of about 38 mm for the fibers comprising 6.7 dtex and 60 mm for the fibers comprising 3.3 dtex. The fibers at the liquid delivery side 6 comprise an average length of 38 mm. For the hydrophilic core 9 the same pulp was used like in example 1.

**table 6**

| layer arrangement | basis weight | layer type | fiber type | linear density |
|---|---|---|---|---|
| liquid acquisition side | 60 g/m² | carded | 50% PET 3.3 dtex | 3.3 dtex |
| | | | 50% PET 6.7 dtex | 6.7 dtex |
| hydrophilic core | 35 g/m² | airlaid | 100% Pulp | |
| liquid delivery side | 27 g/m² | carded | 100% PET | 1.7 dtex |

All examples provide good acquisition and distribution properties, wherein also a transport of body liquids against gravitation is provided especially by capillary forces.

To evaluate the performance of the inventive acquisition and distribution composite 2 the sample according to example 2 was compared to different test samples.

As first test sample a typical construction of an acquisition and distribution material known in the art was provided as market reference according to table 7. The test sample was obtained by disassembling a commercial baby diaper, which has good acquisition and distribution properties but no pronounced distribution of liquid against gravity. The isolated acquisition and distribution layer exposed during disassembly was used for further testing. Analysis of the material revealed a mixture of PET fibers with different linear densities and a typical length of about 60 mm.

**table 7**

| layer arrangement | basis weight | layer type | fiber type | linear density |
|---|---|---|---|---|
| single layer | 50 - 55 g/m² | carded | PET with hydrophilic binder | 3-9 dtex |

The fibers of the market reference comprise a length of about 40 mm to 60 mm. A very fast acquisition and distribution of liquid is observed but the material does not exhibit significant capillary properties.

As a second test sample a tissue material according to table 8 was provided comprising significant capillary properties but a poor acquisition and distribution performance. The typical length of the pulp fibers is about 2.5 mm mm

**table 8**

| layer arrangement | basis weight | layer type | fiber type | linear density |
|---|---|---|---|---|
| single layer | 40 g/m² | creped through-air drying (CTAD) | 100% Pulp | |

As a third test sample ATB 35 nonwoven material consolidated by air through bonding according to table 9 was provided.

**table 9**

| layer arrangement | basis weight | layer type | fiber type | fiber length | linear density |
|---|---|---|---|---|---|
| single layer | 35 g/m² | air through bonding | 34 wt. % PP | 38-40 mm | 10.5 dtex |
| | | | 33 wt. % bicomponent fibers | 40-50 mm | 3.3 dtex |
| | | | 33 wt.% hollow PET | 38 mm | 10.0 dtex |

### Determination of the suction height:

This test procedure measures the traveled distance for transporting a known liquid (distilled water) through a material vertically located within a constant time range.

Colored lines with a graduated scale of 5 mm intervals were drawn across the width of a strip of fabric comprising a length of 220 mm and a width of 25 mm by using a pen filled with water-soluble ink. The dry strip of the fabric was suspended vertically in a frame; with one end clamped onto the frame and the other end dipped in approximately 50 ml of distilled red-colored water. When the water reached the assigned interval of 10 mm, the time started and the first rising distance was recorded. A ruler was placed parallel to the sample for enhancing the accuracy of the test. The wicking heights were recorded at constant time intervals up to a maximum of 30 minutes.

The result of the determination of the suction height is shown in table 10.

**table 10**

| **Product** | **Avg. suction** height [mm] | | | | |
|---|---|---|---|---|---|
| | **60s** | **300 s** | **600 s** | **900 s** | **1800 s** |
| **Example 2** | 61,33 | 86,33 | 95,67 | 100,67 | 105,33 |
| **Market reference (resin bonded)** | 5,97 | 7,67 | 8,00 | 8,00 | 11,00 |
| **Tissue** | 44,00 | 74,67 | 89,00 | 95,83 | 109,33 |
| **ATB 35** | 0 | 0 | 0 | 0 | 0 |

### Liquid transportation test:

This test procedure measures the time taken for a known volume of liquid (simulated urine) to be transported over the edges of a saturated material when applying a pressure gradient, until the amount of liquid is deposited in small reservoirs. The liquid is applied to the surface of the nonwoven facing the skin of the body.

Using the device Lister AC by the company Lenzing Instruments GmbH & Co. (described in test method NWSP 070.3.R1, status of December 2022), a constant volume of 25 ml was applied to the strip of fabric comprising a length of 220 mm and a width of 50 mm and consisting of 9 g/l solution of sodium chloride in demineralized water.

The design of the instrument allows the release of the solution into an electrode plate of 500g total mass, which contains a cavity with a final star shape opening at the bottom, and electrodes are located for detecting the time taken for the liquid to pass through the material, like the strike through method (NWSP 070.3.R1). A stopwatch or chronometer was used to record the exact time when the device detects the solution penetrating the nonwoven fabric.

The preparation of the sample includes coloring the last 10 mm of the edges by using a pen filled with water-soluble ink, and the chronometer helps to visually record the time taken for the solution to penetrate the nonwoven and distribute until it reaches the colored edges, and drops are failing into two aluminum cups located below the edges of the sample.

Two weight measurements are taken, described as it follows:
- Weight of the dry sample before applying the solution.
- Weight of the wet saturated sample.

The results of the liquid transportation test are shown in table 11.

**table 11**

| **Product** | **Avg. Amount of liquid transported [mL]** | **Avg. Period of time for liquid distribution [s]** |
|---|---|---|
| **Example 2** | 7,39 | 31,20 |
| **Market reference (resin bonded)** | 5,88 | 7,33 |
| **Tissue** | 3,04 | 96,15 |
| **ATB 35** | 2,10 | 33,59 |

### Strike through & wetback test:

These test procedures were according to the EDANA test norms NWSO 070.3.R1 (19) and NWSP 80.10.R2 (20). The sample dimension used was changed to 93 mm x 93 mm. The dimension of the blotter paper, which simulates the absorbent pad, was changed as well into 90 mm x 90 mm, so it can be used for wetback test, and it prevents the attraction between filter papers that can disturb the final wetback recordings due to the smaller sample size.

The liquid dose was adjusted according to the sample and filter size, from 22 ml to 18 ml total, where 5 ml was used in strike through the test, and the remaining 13 ml was applied for measuring wetback.

The results of the strike through & wetback test are shown in table 12.

**table 12**

| **Product** | **Strike through time at 5 mL [s]** | **Wetback [g]** |
|---|---|---|
| **Example 2** | 1,12 | 1,00 |
| **Market reference (resin bonded)** | 0,38 | 0,06 |
| **Tissue** | 2,78 | 2,07 |
| **ATB 35** | 1,04 | 0,05 |

### Mechanical properties, Basis weight, thickness and absorption experiments.

The following EDANA test methods were used for the below mentioned properties:
- Basis weight - NWSP 130.1.R0 (15)
- Thickness - NWSP 120.6.R0 (15)
- Tensile strength in machine and cross direction (MDT, CDT) - NWSP 110.4.R0 (15)
- Elongation at break in machine and cross direction (MDE, CDE) - NWSP 110.4.R0 (15)
- Nonwoven fabric absorption - NWSP 010.1.R0

The corresponding test results are shown in table 13.

**table 13**

| **Product** | **Avg. Basis weight [g/m²]** | **Avg. thickness [mm]** | **Avg. MDT [N/5cm]** | **Avg. MDE [%]** | **Avg. CDT [N/5cm]** | **Avg. CDE [%]** | **Absorption [M]** |
|---|---|---|---|---|---|---|---|
| **Example 2** | 58,19 | 0,88 | 76,20 | 40,71 | 11,69 | 273,38 | 1163 |
| **Market reference (resin bonded)** | 51,79 | 1,07 | 44,81 | 21,81 | - | - | 962 |
| **Tissue** | 39,58 | 0,41 | 27,23 | 12,76 | 12,26 | 6,29 | 553 |
| **ATB 35** | 35,66 | 0,59 | 23,79 | 12,06 | 2,95 | 39,51 | 523 |

The provided analysis can be summarizes as follows:
- The example 2 shows higher wetback results compared to a fully air through bonded and resin bonded structures, but it is 2-3 times lower than tissue materials.
- The results obtained via the strike-through method can potentially represent the permeability and indirect pore size distribution, being the resin-bonded material the most permeable, the tissue the less permeable, and example 2 with a behavior in between.
- The capillary pressure of the example 2 is better than a tissue, with 3 times faster liquid transportation, and has sufficient capillary suction to carry the liquid against gravity to the end of the sample (80-150 mm) than compared to common air through bonded and resin bonded nonwovens (0-11 mm).
- Thickness and absorption of the example 2 is comparable to the other technologies, given a defined basis weight.
- The example 2 delivers good mechanical properties, on top of good capillary profile, in comparison to the test samples.

## Claims

1. Acquisition and distribution composite (2) with a liquid acquisition side (5) comprising first nonwoven fibers (7), a hydrophilic core (9) and liquid delivery side (6) comprising second nonwoven (8) fibers, **characterized by** an asymmetric structure with the linear density measured in dtex of the first nonwoven fibers (7) is at least 0.7 dtex greater than that of the second nonwoven fibers (8).

2. Acquisition and distribution composite (2) according to claim 1, **characterized in that** the composite is hydroentangled.

3. Acquisition and distribution composite (2) according to claim 2, **characterized in that** the composite is solely consolidated by hydroentangeling.

4. Acquisition and distribution composite (2) according to one of the claims 1 to 3, **characterized in that** basis weight is between 50 g/cm² and 160 g/cm² (gsm - grams per square centimeter).

5. Acquisition and distribution composite (2) according to one of the claims 1 to 4, **characterized in that** the first nonwoven fibers (7) comprise a linear density of 2 dtex to 8 dtex.

6. Acquisition and distribution composite (2) according to one of the claims 1 to 5, **characterized in that** the composite is formed from 20 to 50 weight percent of the first nonwoven fibers (7).

7. Acquisition and distribution composite according to one of the claims 1 to 6, **characterized in that** the first nonwoven fibers (7) are provided as carded nonwoven.

8. Acquisition and distribution composite according to one of the claims 1 to 7, **characterized in that** the first nonwoven fibers (7) comprise a polyester or long cellulose fibers, especially viscose, as main material component.

9. Acquisition and distribution composite (2) according to one of the claims 1 to 8, **characterized in that** the first nonwoven fibers (7) at least partially comprise a mantle that reduces wettability.

10. Acquisition and distribution composite (2) according to one of the claims 1 to 9, **characterized in that** the first nonwoven fibers (7) are a blend of a first fiber type and a second fiber type, and wherein for the first fiber type and the second fiber type, the linear density is each at least 0.7 dtex greater than that of the second nonwoven fibers.

11. Acquisition and distribution composite (2) according to one of the claims 1 to 10, **characterized in that** short cellulose fibers are provided as hydrophilic core (9).

12. Acquisition and distribution composite (2) according to claim 11, **characterized in that** the short cellulose fibers are at least partially cross-linked.

13. Acquisition and distribution composite (2) according to claim 11 or 12, **characterized in that** the composite is formed from 10 to 50 weight percent of the short cellulose fibers.

14. Acquisition and distribution composite (2) according to one of the claims 1 to 13, **characterized in that** the second nonwoven fibers (8) comprise a linear density of 0.7 dtex to 4 dtex.

15. Acquisition and distribution composite (2) according to one of the claims 1 to 14, **characterized in that** the composite is formed from 20 to 50 weight percent of the second nonwoven fibers.

16. Acquisition and distribution composite (2) according to one of the claims 1 to 15, **characterized in that** the second nonwoven fibers (8) are provided as carded nonwoven.

17. Acquisition and distribution composite (2) according to one of the claims 1 to 16, **characterized in that** the second nonwoven fibers (8) comprise a polyester or long cellulose fibers, especially viscose, as main material component.

18. Absorbent hygiene article comprising an acquisition and distribution composite (2) according to one of the claims 1 to 17, a backsheet (4) and an absorbent core (3) between the backsheet (4) and the acquisition and distribution composite (2).

19. Absorbent hygiene article according to claim 18, wherein the acquisition and distribution composite (2) is provided between the absorbent core (3) and a permeable topsheet (1).

20. Method to produce an acquisition and distribution composite (2) preferably according to one of the claims 1 to 17 comprising:
a. providing a first carded nonwoven (11) of first nonwoven fibers (7),
b. providing a second carded nonwoven (12) of second nonwoven fibers (8),
c. providing an airlaid hydrophilic core (9) comprising short cellulose fibers after providing one of the first and second carded nonwoven (11, 12) and before providing the other of the first and second carded nonwoven (12, 11),
d. hydroentangeling the three layer structure formed by the hydrophilic core and the first and second carded nonwoven to form the acquisition and distribution composite,
wherein the linear density measured in dtex of the first nonwoven fibers (7) is at least 0.7 dtex greater than that of the second nonwoven fibers (8).

21. Method according to claim 20, wherein hydroentangeling is provided on smooth surfaces with waterjets from both sides of the acquisition and distribution composite (2).

22. Method according claim 20 or 21, wherein after hydroentangeling the acquisition and distribution composite (2) is dried and wound on a roll (15) without further bonding, consolidation or structuring.
